# EUROPÄISCHE PATENTSCHRIFT

(11) **EP 3 552 636 B1**
(45) Veröffentlichungstag und Bekanntmachung des Hinweises auf die Patenterteilung: **21.12.2022**
(21) Anmeldenummer: 19167973.7
(22) Anmeldetag: 08.04.2019
(51) Int. Cl.: A61M 1/36, A61M 39/28

(54) **ZUGANGSKANÜLE MIT SPERRVORRICHTUNG**
ACCESS CANNULA WITH BLOCKING DEVICE
CANULE D'ACCÈS POURVUE DE DISPOSITIF DE VERROUILLAGE

(30) Priorität: 09.04.2018 DE 102018108293
(43) Veröffentlichungstag der Anmeldung: 16.10.2019
(73) Patentinhaber: B. Braun Avitum AG, 34212 Melsungen (DE)
(72) Erfinder: RITTER, Kai-Uwe, 34212 Melsungen (DE)
(74) Vertreter: Winter, Brandl - Partnerschaft mbB

(56) Entgegenhaltungen:
- WO-A1-97/11296
- US-A- 3 029 059
- US-A1- 2006 016 478
- US-A1- 2008 281 276

## Beschreibung

Bei der vorliegenden Erfindung handelt es sich um eine Zugangskanüle, insbesondere eine Venenverweilkanüle, mit einem Verweilschlauch, welcher dazu konfiguriert ist, während einer Behandlung, insbesondere einer Dialysebehandlung, als Zugang zu dienen, wobei eine Sperrvorrichtung dazu vorgesehen ist, den Verweilschlauch in einer Ruhelage abgeknickt zu halten.

### Hintergrund der Erfindung

Dialyse, insbesondere Hämodialyse, Hämofiltration und Hämodiafiltration, stellt die häufigste Behandlungsform bei Nierenversagen dar und erfordert häufige und regelmäßige Behandlungen für einen Patienten, welche sowohl in einer Klinik durchgeführt werden können als auch zu Hause von dem Patienten selbst.

Bei einer Dialysebehandlung werden mittels einer arteriellen Einstecknadel ein arterieller Zugang und mittels einer venösen Einstecknadel ein venöser Zugang gelegt, um den Patienten an eine Dialysemaschine anzuschließen. Hierfür werden Venenverweilkanülen oder -katheter eingesetzt, bei welchen ein Kunststoffschlauch oder Verweilschlauch über eine Einstecknadel oder Metallkanüle in ein Patientengefäß eingeführt wird und nach Entfernen der Einstecknadel als Zugang in der Vene verbleibt. Derartige Venenverweilkanülen sind aus dem Stand der Technik hinreichend bekannt.

Wenn sich bei einer Dialysebehandlung der venöse Zugang löst und aus dem Gefäß rutscht, wird dies als venöse Nadeldislokation (VND) bezeichnet, was eine kritische Komplikation und Gefahr für den Patienten darstellt. In einem solchen Fall fließt Blut, welches die Dialysemaschine durchflossen hat, nicht zurück in den Kreislauf des Patienten, sondern wird in die Umgebung gepumpt, insbesondere durch eine Blutpumpe der Behandlungsmaschine. Ein durchschnittlicher Patient kann bei einer VND innerhalb von 5 Minuten bis zu 50% seines Gesamtblutvolumens verlieren und es reichen bereits zwei Minuten, bis Vitalzeichen eines Patienten signifikant beeinträchtigt sind. Folglich ist es essentiell wichtig, eine VND möglichst schnell zu erkennen. Je nach Reaktionszeit liegen die Folgen bei einem relativ geringen Blutverlust, erhöhter Infektionsgefahr, irreversiblen Hirnschäden bis hin zum Tod. Neben den physischen und psychischen Folgen für den Patienten fallen durch benötigte Blutkonserven und Folgebehandlungen auch hohe Kosten an. Insbesondere bei Heimdialyse und nächtlichen Behandlungen, wo die Patienten zumeist schlafen, sind VND kritisch, da der Patient nicht in der Lage ist, die VND selbst zu bemerken und möglicherweise nicht rechtzeitig reagieren und auf sich aufmerksam machen kann.

Eine besondere Herausforderung besteht darin, dass eine VND nahezu jederzeit auftreten kann. Zwar sind einige Risikofaktoren bekannt, wie beispielsweise Rastlosigkeit oder mentale Beeinträchtigung der Patienten sowie das Auftreten von Muskelkrämpfen während der Behandlung, jedoch treten VND auch unter Idealbedingungen auf und sind somit kaum vorhersagbar.

### Stand der Technik

Um die Gefahr einer VND zu verringern, gibt es Richtlinien zu der Art und Weise, wie ein Zugang gelegt und befestigt werden soll. Ferner existieren Lösungen, welche ein Auftreten einer VND erkennen und einen Alarm auslösen, sowie ggf. die Blutpumpe der Behandlungsmaschine anhalten können.

Eine solche Lösung ist eine Drucküberwachung an der venösen Leitung bzw. an dem venösen Zugang, was in Dialysemaschinen standardmäßig vorgesehen ist. Die Überwachung des venösen Druckverlaufs gilt jedoch als notorisch unsicher, unter anderem weil der Druck abhängig von einer Vielzahl von Faktoren stark variieren kann. Häufig wird eine VND nur verzögert erkannt, weshalb der Patient bereits bis zum Auslösen des Alarms eine signifikante Menge Blut verloren haben kann. Ferner kommt es vor, dass beispielsweise aufgrund von Strömungswiderständen an der Nadel oder deren Befestigungsmaterial sowie bei unvollständigem Lösen der Nadel der Druckabfall so gering ist, dass dieser nicht als VND erkannt wird und gar kein Alarm ausgelöst wird.

Eine weitere Lösung besteht in der Erkennung einer Leckage durch an dem Zugang angebrachte Leckage- oder Feuchtigkeitssensoren, welche einen Blutverlust in die Umgebung überwachen, wie zum Beispiel durch VenAcc von Fresenius Medical Care und Redsense von Redsense Medical, welche ein Einweg-Sensorpad aufweisen, das zusammen mit der Nadel aufgeklebt wird und bereits geringe Mengen austretender Flüssigkeiten erkennt. Allerdings sind diese Vorrichtungen mit zusätzlichen Kosten verbunden, da hierfür teure Zusatzgeräte und Einwegteile benötigt werden und menschliche Fehler hierdurch nicht ausgeschlossen werden können.

US 2008/0281276 A1 zeigt ein auf Haut aufklebbares Infusionsset, an dem eine Zugangskanüle aus einer Formgedächtnislegierung angeordnet ist, welche unter Körperwärme gestreckt werden kann und bei Raumtemperatur in die vorgeformte, geknickte Stellung zurückkehrt, um einen Fluss durch die Kanüle zu unterbrechen.

Die bekannten Lösungen ermöglichen somit keine hundertprozentig sichere Erkennung von VND. Da außerdem durch Fehlanwendung bzw. Fehlbedienung oder zu eng gesetzte Grenzwerte die Wahrscheinlichkeit für Fehlalarme hoch ist, werden venöse Alarme häufig einfach weggedrückt, sodass trotz Anschlagen eines Alarms eine VND ggf. nicht rechtzeitig erkannt wird.

### Kurzbeschreibung der Erfindung

Die Aufgabe der vorliegenden Erfindung besteht folglich darin, ein System zur Verfügung zu stellen, welches u.a. die vorstehend genannten Nachteile bestehender Systeme behebt und insbesondere eine zuverlässige, schnelle Erkennung von VND und eine möglichst schnelle, zuverlässige Unterbrechung des Blutflusses ermöglicht. Ferner besteht die Aufgabe der Erfindung darin, ein derartiges System zur Verfügung zu stellen, welches keinen apparativen Mehraufwand erfordert, sowie kostengünstig und einfach zu handhaben ist.

Die Erfindung wird durch die Merkmale des unabhängigen Anspruchs 1 definiert.

Diese Aufgabe wird gelöst durch eine Zugangskanüle, insbesondere eine Venenverweilkanüle, mit einem Verweilschlauch, welcher dazu konfiguriert ist, während einer Behandlung, insbesondere einer Dialysebehandlung, als ein Zugang in einen Patientenkörper zu dienen. Dabei ist eine Sperrvorrichtung vorgesehen, welche dazu konfiguriert ist, in einer Ruhelage (in welcher der Verweilschlauch nicht oder nicht korrekt im Patientenkörper appliziert ist) den Verweilschlauch abgeknickt zu halten. Die Sperrvorrichtung hat ein Federelement, welches mit einem ersten Ende an einer ersten Position des Verweilschlauchs angeordnet ist und welches mit einem zweiten Ende an einer zweiten Position mit dem Verweilschlauch verbunden oder verbindbar ist. Dabei ist die zweite Position entlang einer Längsachse des Verweilschlauchs mit einem Abstand von der ersten Position entfernt. Ferner weist die erfindungsgemäße Zugangskanüle einen Befestigungssockel oder einem Befestigungspad zum klebenden Befestigen des Verweilschlauchs an Patientenhaut auf, welches an der ersten Position oder der zweiten Position vorgesehen ist.

Die erfindungsgemäße Zugangskanüle ist insbesondere dazu vorgesehen, um den venösen Zugang zu legen, kann jedoch gleichermaßen auch für den arteriellen Zugang verwendet werden. Ferner soll die Erfindung nicht auf Dialysebehandlungen beschränkt sein. Beispielsweise ist auch ein Einsatz als Zugang für eine Infusion denkbar, wo eine erfindungsgemäße Zugangskanüle ermöglicht, bei einer Nadeldislokation ein Auslaufen von Medikamenten etc. zu verhindern. Dies ist insbesondere bei Medikamenten zur onkologischen Behandlung relevant.

Die Ruhelage, in welcher der Verweilschlauch vorzugsweise an einer Soll-Knickstelle abgeknickt gehalten wird, entspricht einer Lage, in welcher eine Kraft, die die Sperrvorrichtung auf den Verweilschlauch ausübt, die elastische Rückstellkraft des Verweilschlauchs und ggf. ein in dem Verweilschlauch vorliegender Druck einander ausgleichen.

Um als Zugang dienen zu können, muss die Zugangskanüle mit dem Verweilschlauch in einer im Wesentlichen oder nahe einer gestreckten Lage entgegen der Kraft der Sperrvorrichtung gehalten werden, erfindungsgemäß durch klebende Befestigung an Patientenhaut durch einen an der Zugangskanüle vorgesehenen Befestigungssockel oder -pad, sodass ein möglichst widerstandsloser Blutfluss durch den Schlauch möglich ist. Da beim Legen des Zugangs wie bereits vorstehend beschrieben der Verweilschlauch über eine Einstecknadel geschoben und so in die Vene einführt wird, folgt der Verweilschlauch dabei der Geometrie der Einstecknadel, d.h. er ist gestreckt. Anschließend kann der Verweilschlauch in dieser Lage am Patientenkörper befestigt werden und die Einstecknadel abgezogen werden. Somit wird während des standartmäßigen Legens des Zugangs der Schlauch durch die Nadel gestreckt gehalten, wodurch hierfür keine zusätzlichen Teile oder Bewegungen/Handhabung benötigt werden und der Einsatz einer erfindungsgemäßen Zugangskanüle im Vergleich mit einer derzeit gängigen Zugangskanüle keinen Mehraufwand für das medizinische Personal bedeutet.

Wenn der Verweilschlauch aus der Vene des Patienten rutscht, d.h. wenn es zu einer Nadeldislokation kommt, dann löst sich auch die Anbringung/Befestigung der Zugangskanüle an dem Patienten. Folglich wird der Verweilschlauch nicht mehr in gestreckter Lage gehalten und durch die Sperrvorrichtung abgeknickt. Dies unterbricht oder hemmt den Blutfluss durch das Behandlungssystem, z.B. die Dialysemaschine, sodass kein oder wenig Blut aus dem Verweilschlauch ausströmen kann. Insbesondere bei einer VND, also einer Nadeldislokation an dem venösen Zugang, verhindert dies im günstigsten Fall, dass Blut aus dem arteriellen Zugang durch das Behandlungssystem und aus dem gelösten venösen Zugang in die Umgebung gepumpt wird.

Das Absperren des Blutflusses beispielsweise in einem extrakorporalen Blutkreis einer Dialysemaschine führt bei einer Sperrung des venösen Zugangs zu einem erhöhten Blutdruck stromaufwärts des Knicks sowie bei einer Sperrung des arteriellen Zugangs zu einem abfallenden Blutdruck stromabwärts des Knicks. Diese Druckänderung erfolgt schnell und ist groß genug, dass sie sicher von dem in dem Behandlungssystem, insbesondere der Dialysemaschine, standardmäßig vorgesehenen Drucküberwachungssystem erfasst werden und eine Reaktion eingeleitet werden kann, d.h. ein Alarm kann unmittelbar ausgegeben und ggf. eine Blutpumpe abgeschaltet bzw. angehalten werden. Vorteilhaft ist hierbei insbesondere, dass ein Alarm zwar weggedrückt werden kann, dieser aufgrund der Sperrung des Blutflusses jedoch sofort wieder anschlagen würde.

Es ist zu beachten, dass die Kraft, mit welcher die Sperrvorrichtung den Schlauch abgeknickt hält, im Falle einer VND nicht nur entgegen der elastischen Rückstellkraft des Schlauchs wirkt, sondern ggf. auch entgegen des durch die Blutpumpe aufgebauten Druckes. Entsprechend ist die Sperrvorrichtung einerseits abhängig von einer Förderleistung der eingesetzten Blutpumpe derart eingestellt bzw. einstellbar, dass ein Wiederöffnen des Verweilschlauchs aufgrund des steigenden Blutdrucks verhindert oder zumindest für eine vorbestimmte Zeit verzögert wird. Andererseits muss die Sperrvorrichtung derart eingestellt sein, dass im Fall eines klebend an der Patientenhaut gestreckt angebrachten Verweilschlauchs diese Klebung nicht durch die Sperrvorrichtung selbst gelöst werden kann, was zu einer Unterbrechung der Behandlung führen würde und schlimmstenfalls sogar eine VND auslösen könnte.

Nach einem weiteren bevorzugten Aspekt der Erfindung sind der Abstand und das Federelement derart eingestellt, dass das Federelement gespannt ist, wenn der Verweilschlauch in einer gestreckten Lage gehalten wird.

Das Federelement dient dazu, eine (Biege-) Kraft/Federkraft auf den Verweilschlauch auszuüben, wodurch dieser abgeknickt werden kann. Die Federkraft entspricht hierbei der Kraft der Sperrvorrichtung. Das Federelement ist bedingt durch den Abstand der ersten und der zweiten Position und/oder durch die Rückstellkraft des Verweilschlauchs auch in der Ruhelage geringfügig vorgespannt und ist in gestreckter Lage über die Vorspannung der Ruhelage hinaus zusätzlich gespannt. Grundsätzlich ist es auch denkbar, mehrere Federelemente vorzusehen. Der Abstand kann durch die Positionen der Verbindungen der Sperrvorrichtung mit dem Verweilschlauch bestimmt sein oder bedingt durch andere strukturelle Parameter, wie die Länge der Feder, bestimmt sein. Die erste Position kann sowohl distal als auch proximal von der zweiten Position liegen. Die erste Position oder die zweite Position kann die gleiche Position sein, an welcher der Befestigungssockel oder das Befestigungspad zum klebenden Befestigen des Verweilschlauchs an der Patientenhaut vorgesehen ist. Der Abstand ist derart eingestellt, dass die auf den Verweilschlauch ausgeübte Federkraft eine vorbestimmte Federkraft ist, welche wie vorstehend beschrieben beispielsweise von der Förderleistung der Blutpumpe und/oder der gestreckten Befestigung des Verweilschlauchs abhängig ist. Der Abstand ist vorzugsweise ein fest eingestellter Abstand, welcher u.a. abhängig von der Länge des Federelements ist, oder ist alternativ unmittelbar durch Parameter des Federelements bestimmt. Es ist jedoch auch denkbar, dass der Abstand der ersten und der zweiten Position einstellbar und sicherbar ist, indem eine der beiden Positionen gegenüber der anderen veränderbar ist. Dies ist beispielsweise dadurch realisierbar, dass ein Element, welches das Federelement mit dem Verweilschlauch verbindet, entlang des Verweilschlauchs verschiebbar ist, oder dass entlang des Verweilschlauchs mehrere Elemente zum Halten/Anschließen eines der Enden des Federelements vorgesehen sind und das entsprechende Ende des Federelements umgesetzt bzw. umgehakt werden kann, d.h. je nach gewünschter Kraft/Federkraft des Sperrelements das Federelement von unterschiedlichen Elementen gehalten werden kann.

Nach einem bevorzugten Aspekt der Erfindung übt das Federelement an der zweiten Position eine Federkraft auf den Verweilschlauch aus, welche zumindest anteilig senkrecht zu der Längsachse des Verweilschlauchs wirkt, wenn das Federelement gespannt ist.

Dies ermöglicht es, den Verweilschlauch mit einem relativ geringen Kraftaufwand abzuknicken, verglichen mit einem Fall, in welchem der Verweilschlauch nur in Längsrichtung mit Kraft beaufschlagt würde und ähnlich einem druckbelasteten Knickstab nachgeben müsste. Die durch das Federelement ausgeübte Federkraft kann ausschließlich senkrecht zu der Längsachse des Verweilschlauchs wirken, sie kann jedoch stattdessen auch sowohl aus einer senkrechten Kraftkomponente als auch aus einer Kraftkomponente parallel zu der Längsachse des Verweilschlauchs zusammengesetzt sein. Da das Federelement an einer ersten Position des Verweilschlauchs angebracht ist, bewirkt dies ein Moment, welches auf den Verweilschlauch wirkt und zu einem Abknicken führt.

Nach einem bevorzugten Aspekt der Erfindung ist der Verweilschlauch durch eine Federkraft des Federelements vorzugsweise an einer Soll-Knickstelle abgeknickt, wenn der Verweilschlauch nicht in einer gestreckten Lage gehalten wird.

In anderen Worten ist die Federkraft des Federelements derart eingestellt, dass der Verweilschlauch stets abgeknickt ist, es sei denn eine gestreckte Lage wird gezielt hergestellt. Auch falls beispielsweise eine VND auftritt und dabei ein Arm des Patienten auf dem Verweilschlauch aufliegen würde, tendiert dieser eher dazu, sich wegzudrehen, als sich zu strecken, sodass der Verweilschlauch seine abgeknickte Stellung oder seine Ruhestellung beibehält. Eine gestreckte Lage des Verweilschlauchs sollte also nur hergestellt werden können, wenn gezielt beide Positionen, an welchen die Enden des Federelements befestigt sind, derart gehalten und ggf. fixiert sind, dass der Schlauch gestreckt ist.

Nach einem weiteren bevorzugten Aspekt der Erfindung hat die Sperrvorrichtung einen Basisabschnitt, welcher das erste Ende des Federelements mit dem Verweilschlauch direkt oder indirekt verbindet oder verbindbar macht, und einen Anschlussabschnitt, welcher das zweite Ende des Federelements mit dem Verweilschlauch verbindet oder verbindbar macht.

Im dem Fall, in dem die erste Position und die zweite Position zueinander einstellbar/veränderbar sind, kann hierbei der Basisabschnitt und/oder der Anschlussabschnitt auf dem Verweilschlauch verschiebbar und ggf. sicherbar sein oder mehrere unterschiedlich positionierte Elemente aufweisen, welche dazu angepasst sind, ein Ende des Federelements zu halten. Ein verschiebbarer Basis- oder Anschlussabschnitt kann beispielsweise eine geschlossene oder geschlitzte, vorzugsweise aufklipsbare Hülse oder ein Ring, z.B. ein Gummiring, sein, welche bzw. welcher ggf. entlang des Schlauchs verschoben und ggf. festgeklemmt werden kann. Elemente, welche ein Ende des Federelements halten, können beispielsweise Haken, Ösen, Nuten oder Hinterschnitte sein. Falls der Abstand der beiden Positionen zueinander nicht einstellbar ist, können der Basisabschnitt und/oder der Anschlussabschnitt fest mit dem Verweilschlauch verbunden oder verbindbar sein. Ferner kann der Basisabschnitt beliebig zu der übrigen Zugangskanüle ausgerichtet sein. Beispielsweise kann die Seite des Basisabschnitts, an welcher das Federelement angeschlossen oder befestigt ist (hier als "oben bezeichnet"), der Seite, an welcher sich der erfindungsgemäße Befestigungssockel oder -pad der Zugangskanüle befindet (hier als "unten" bezeichnet), radial gegenüberliegen. Alternativ kann der Basisabschnitt zu der Zugangskanüle jedoch in einem beliebigen Winkel ausgerichtet sein, beispielsweise gegenüber der Ausrichtung des vorstehenden Beispiels um 90° verdreht, sodass das Federelement sich "seitlich" des Basisabschnitts befindet und die Seite, an welcher sich ggf. der Befestigungssockel oder -pad der Zugangskanüle befindet, "unten" liegt.

Nach einem bevorzugten Aspekt der Erfindung sind zumindest der Basisabschnitt und/oder der Anschlussabschnitt, vorzugsweise die gesamte Sperrvorrichtung inklusive dem Federelement, mit dem Verweilschlauch einstückig ausgebildet oder als ein von dem Verweilschlauch unterschiedliches Bauteil einstückig ausgebildet, vorzugsweise als Spritzgussteil.

Auf diese Weise ist es möglich, die erfindungsgemäße Zugangskanüle kostengünstig als Einwegteil zu fertigen. Falls die Sperrvorrichtung getrennt von dem Verweilschlauch gefertigt bzw. spritzgegossen ist, kann diese in einem weiteren Fertigungsschritt mit dem Verweilschlauch verbunden, z.B. geklebt, werden. Dabei kann ggf. die Sperrvorrichtung mit anderen Teilen der Zugangskanüle wie z.B. dem Befestigungssockel oder -pad einstückig gefertigt sein. Auf diese Weise kann der Verweilschlauch ein Zukaufteil sein. Dies ist ebenfalls möglich, wenn die Sperrvorrichtung oder Teile der Sperrvorrichtung direkt auf den Verweilschlauch aufgespritzt werden und somit mit diesem einstückig ausgebildet sind.

Nach einem bevorzugten Aspekt der Erfindung ist das Federelement als Biegefeder ausgebildet.

Dies stellt eine Ausführungsform der Erfindung dar und ist insbesondere vorteilhaft, da hierbei die gesamte Sperrvorrichtung und ggf. sogar die gesamte Zugangskanüle inklusive dem Verweilschlauch einstückig fertigbar ist, insbesondere spritzgegossen werden kann und somit besonders günstig herzustellen ist. Die Biegefeder kann zum Beispiel als Blattfeder, als Biegebalken oder als Biegestab ausgebildet sein. Um die Sperrvorrichtung einsatzfertig zu machen oder in die Ruhelage zu bringen, kann die Biegefeder in einem weiteren Fertigungsschritt gebogen (gespannt) werden. Hierfür kann beispielsweise der Anschlussabschnitt an der zweiten Position mit dem Verweilschlauch verbunden werden. Vorzugsweise kann dabei der Anschlussabschnitt gemäß einem der vorstehend beschriebenen Aspekte der Erfindung entlang der Längsachse des Verweilschlauchs frei verschiebbar sein, sodass die Position des Anschlussabschnitts (die zweite Position) und deren Abstand zu der ersten Position durch die Länge der Biegefeder vorbestimmt ist. Der Anschlussabschnitt kann sowohl gelenkig (beispielsweise um eine Achse senkrecht zu der Längsachse des Verweilschlauchs drehbar) und/oder verschiebbar mit dem Verweilschlauch verbunden oder verbindbar sein, als auch fest mit dem Verweilschlauch verbunden oder verbindbar sein, z.B. durch Kleben. Eine drehfeste Verbindung zwischen der Biegefeder und dem Verweilschlauch führt dazu, dass der Winkel, in welchem die Federkraft auf den Verweilschlauch wirkt, stets gleich ist. Insbesondere, falls die Biegefeder mit der Längsachse des Verweilschlauchs an der Verbindungsstelle zwischen der Biegefeder und dem Verweilschlauch einen starren, rechten Winkel bildet, hat die Federkraft der Biegefeder genau genommen keine Komponente, welche senkrecht zu der Längsachse des Verweilschlauchs ist. Jedoch führt das Moment, welches durch die Biegefeder auf den Verweilschlauch aufgebracht wird, direkt dazu, dass ein Teil des Verweilschlauchs sich in eine Richtung senkrecht zu der Längsachse des Verweilschlauchs bewegt, wenn er sich aus seiner gestreckten Lage herausbewegt. Das führt zum Abknicken des Verweilschlauchs. Im Kontext dieser Erfindung kann dies als senkrecht zu der Längsachse des Verweilschlauchs wirkende Kraft gemäß einem der vorstehend beschriebenen Aspekte der Erfindung angesehen werden.

Nach einem bevorzugten Aspekt der Erfindung ist das Federelement als Zugfeder, vorzugsweise als elastisches Band, ausgebildet.

Dies stellt eine weitere Ausführungsform der Erfindung dar. Hierbei wird die Zugfeder an ihrem einen Ende an dem Basisabschnitt gehalten oder eingehakt und an ihrem zweiten Ende an dem Anschlussabschnitt gehalten oder eingehakt. Dabei definiert der Basisabschnitt einen radialen Abstand des ersten Endes des Federelements von der Längsachse des Verweilschlauchs. Ggf. hat der Basisabschnitt hierfür einen stabförmigen Teil oder ist nockenförmig ausgebildet oder hat einen entsprechend großen Gesamtdurchmesser, es kann jedoch gleichermaßen jede sich ausgehend von der Längsachse des Verweilschlauchs radial nach außen erstreckende Struktur genutzt werden, um den radialen Abstand des ersten Endes des Federelements von der Längsachse des Verweilschlauchs festzulegen. Die Zugfeder kann anstelle eines elastischen Bandes beispielsweise auch eine Spiralfeder oder Ähnliches sein. Der Basisabschnitt und der Anschlussabschnitt können einstückig mit dem Verweilschlauch gefertigt, insbesondere an diesen angegossen werden und sind unmittelbar mit diesem verbunden. Bevorzugt ist die zweite Position des Verweilschlauchs bzw. der Anschlussabschnitt entlang der Längsachse des Verweilschlauchs verschiebbar, wobei ein Minimalabstand von der ersten Position bzw. dem Basisabschnitt beispielsweise durch einen Anschlag definiert ist. Die Kraft/Federkraft der Sperrvorrichtung kann ggf. dadurch eingestellt werden, dass das Federelement gegen ein anderes unterschiedlich starkes Federelement ausgetauscht wird und/oder dass der Anschlussabschnitt und/oder der Basisabschnitt gemäß einem der vorstehend beschriebenen Aspekte der Erfindung mehrere auswählbare, unterschiedlich positionierte Elemente zum Halten eines der Enden der Feder aufweisen.

Nach einem bevorzugten Aspekt der Erfindung ist der Basisabschnitt derart ausgebildet, dass das erste Ende des als Zugfeder ausgebildeten Federelements an dem Basisabschnitt mit einem vorbestimmten radialen Abstand von der Längsachse des Verweilschlauchs verbunden oder verbindbar ist.

Der Winkel der Zugfeder zu der Längsachse des Verweilschlauchs ist nach diesem Aspekt der Erfindung abhängig von dem vorbestimmten radialen Abstand. Folglich ist auch die senkrechte Kraftkomponente der Federkraft, welche senkrecht zu der Längsachse des Verweilschlauchs wirkt, abhängig von dem vorbestimmten radialen Abstand. Hierbei ist der radiale Abstand klein genug gewählt, dass der Basisabschnitt nicht im Weg ist und die Gefahr, daran hängen zu bleiben gering ist, aber groß genug, dass die senkrechte Kraftkomponente ausreichend groß ist, um den Verweilschlauch erfindungsgemäß abzuknicken.

Insbesondere und bevorzugt wird die Aufgabe gelöst durch eine Zugangskanüle bei welcher der Verweilschlauch an der ersten Position einen Befestigungssockel oder ein Befestigungspad aufweist, das zur vorzugsweise klebenden Fixierung des Verweilschlauchs an einer Patientenoberfläche, vorzugsweise Patientenhaut ausgebildet ist und an dem das Federelement an dessen erstem Ende fixiert oder gehalten ist, und der Verweilschlauch an der zweiten Position ein Anschlusselement oder einen Anschlussabschnitt aufweist, an dem das Federelement an dessen zweitem Ende fixiert oder gehalten ist, wobei der Verweilschlauch axial zwischen der ersten und zweiten Position mit einer Soll-Knickstelle ausgebildet ist und das Federelement entweder als Zug- oder Druckfeder, vorzugsweise in Form eines elastischen Bands oder einer Schraubenfeder, oder als Biegefeder, vorzugsweise in Form einer Blattfeder oder eines Federbalkens, ausgebildet ist, die zwischen der ersten und zweiten Position derart eingebaut und/oder ausgerichtet ist, dass das Federelement in einer gespannten Lage außerhalb der Ruhelage eine Kraft auf den Verweilschlauch an der zweiten Position ausübt, die zumindest teilweise senkrecht zur Längsachse des Verweilschlauchs wirkt, um diesen aus einer wenig oder nicht geknickten Stellung in die abgeknickte Stellung vorzuspannen.

### Fiqurenbeschreibunq

Die Erfindung wird im Folgenden anhand bevorzugter Ausführungsformen beschrieben, wobei Merkmale verschiedener Ausführungsformen untereinander ausgetauscht und/oder kombiniert werden können. Es versteht sich, dass Einzelheiten der beschriebenen bevorzugten Ausführungsformen die Erfindung als solche nicht beschränken und sich für den Fachmann naheliegend verschiedenartige Änderungen, Modifikationen und/oder Äquivalente ergeben können, die als solche allesamt im Rahmen des durch die beigefügten Ansprüche definierten Schutzbereichs der Erfindung liegen. Ferner werden in der nachfolgenden Beschreibung der bevorzugten Ausführungsformen gleiche Bezugszeichen für einander entsprechende Merkmale verwendet, welche ggf. nur einmal beschrieben werden, jedoch in jeder der vorgestellten Ausführungsformen vorgesehen sein können.
Fig. 1 zeigt eine Sperrvorrichtung nach einer ersten Ausführungsform der Erfindung in entspannter Lage,
Fig. 2 zeigt eine Zugangskanüle nach der ersten Ausführungsform, welche auf einer Einstecknadel geführt ist,
Fig. 3 zeigt eine Zugangskanüle nach der ersten Ausführungsform, welche in Ruhelage ist,
Fig. 4 zeigt einen Teil einer Zugangskanüle nach der zweiten Ausführungsform,
Fig. 5 zeigt eine Zugangskanüle nach einer zweiten Ausführungsform, welche auf einer Einstecknadel geführt ist,
Fig. 6 zeigt eine Zugangskanüle nach der zweiten Ausführungsform, welche in Ruhelage ist.

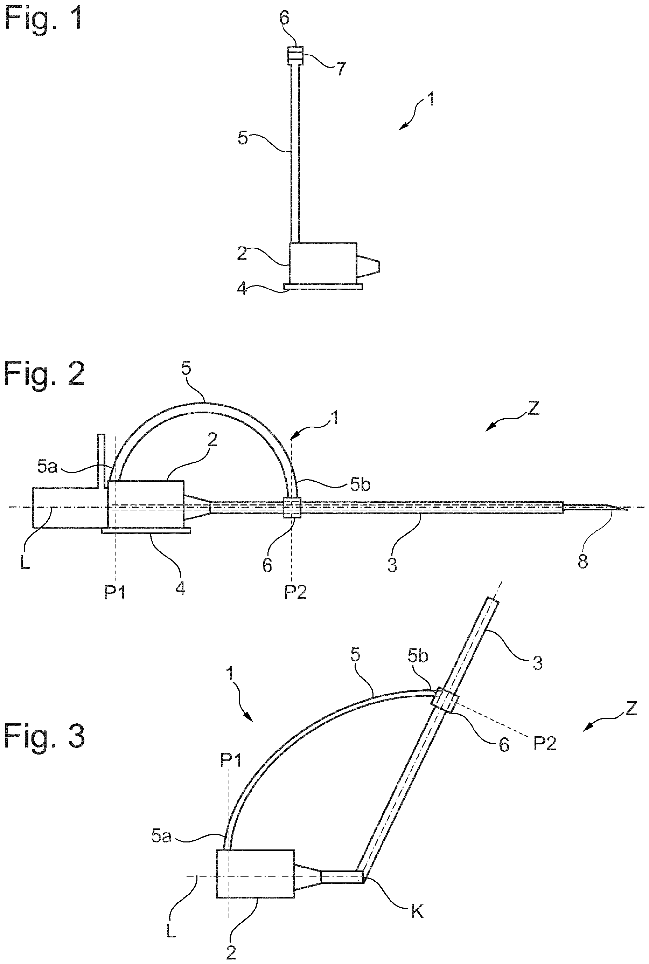

Fig. 1 zeigt eine Sperrvorrichtung 1 einer Zugangskanüle Z nach einer ersten Ausführungsform der Erfindung. Dabei ist die gesamte Sperrvorrichtung 1 einstückig ausgebildet, wobei sämtliche ihrer Elemente aus dem gleichen Material gefertigt sein können, vorzugsweise durch Spritzgießen. Die Sperrvorrichtung 1 hat einen hülsenförmigen Basisabschnitt 2, welcher dazu vorgesehen ist, an einem Verweilschlauch 3 montiert bzw. darauf aufgesteckt zu werden. Der Basisabschnitt 2 ist unmittelbar und einstückig mit einem Befestigungssockel 4 der Zugangskanüle Z verbunden, welcher dazu dient, die Zugangskanüle Z für eine Behandlung an einem Patienten, genauer an einem Hautabschnitt des Patienten, vorzugsweise klebend zu fixieren. Ferner weist die Sperrvorrichtung 1 ein in der abgebildeten Lage entspanntes Federelement 5 in Form einer Biegefeder auf, welches unmittelbar und einstückig an dem Basisabschnitt 2 angeschlossen ist. Dabei ist das Federelement 5 auf der dem Befestigungssockel 4 radial gegenüberliegenden Seite des Basisabschnitts 2 angeordnet. Ferner weist die Sperrvorrichtung 1 einen Anschlussabschnitt 6 auf, welcher unmittelbar und einstückig an das Federelement 5 angeschlossen ist. Der Anschlussabschnitt 6 ist hülsenförmig mit einem Aufnahmeloch 7 zum Aufnehmen des Verweilschlauchs 3 ausgebildet.

Fig. 2 zeigt die Zugangskanüle Z nach der ersten Ausführungsform in einem fertig montierten Zustand. Das heißt, die Zugangskanüle Z befindet sich in dem Zustand, in welchem sie dafür genutzt werden kann, einen Zugang für eine Behandlung zu legen. Hierbei sitzt der Verweilschlauch 3 auf einer Einstecknadel 8, welche dazu dient, beim Legen des Zugangs Patientengewebe wie dessen Haut und Gefäße, zu punktieren. Die vorstehend beschriebene Sperrvorrichtung 1 gemäß Fig. 1 ist an dem Verweilschlauch 3 montiert. Die Biegefeder als das Federelement 5 ist gebogen und gespannt und der hülsenförmige Anschlussabschnitt 6 ist an dem Verweilschlauch 3 montiert, indem der Verweilschlauch 3 durch das Aufnahmeloch 7 des Anschlussabschnitts 6 geführt wurde. Da das Federelement 5 eine entspannte, gestreckte Lage anstrebt, übt es hierbei eine Kraft bzw. ein Moment mit einer Komponente, welche senkrecht zu einer Längsachse L des Verweilschlauchs 3 steht, auf den Verweilschlauch 3 aus, sodass hierdurch ggf. ein Abknicken des Verweilschlauchs 3 ausgelöst wird. Der Anschlussabschnitt 6 ist auf dem Verweilschlauch 3 frei verschiebbar, sodass der Anschlussabschnitt 6 sich beim Montieren selbst entlang des Verweilschlauchs 3 ausrichtet und kein zusätzliches Einstellen des Federelements 5 notwendig ist. Da der Verweilschlauch 3 auf der Einstecknadel 8 sitzt, welche die Form einer langen, geraden, dünnen Hülse hat, ist die Form des Verweilschlauchs 3 der Form der Einstecknadel 8 angepasst, d.h. der Verweilschlauch 3 wird durch die Einstecknadel 8 in einer gestreckten Lage gehalten.

Beim Legen des Zugangs wird die Einstecknadel 8 der Zugangskanüle Z in das Gefäß des Patienten eingeführt. Anschließend wird der Verweilschlauch 3 über die Einstecknadel 8 in das Gefäß eingeschoben und die Zugangskanüle Z wird an der Haut des Patienten fixiert. Anschließend wird die Einstecknadel 8 abgezogen und entfernt, während der Verweilschlauch 3 in dem Gefäß des Patienten verbleibt und während der Behandlung als Zugang dient.

Fig. 3 zeigt die erfindungsgemäße Zugangskanüle Z nach der ersten Ausführungsform in einer Ruhelage, welche dann vorliegt, wenn der Verweilschlauch 3 nicht in gestreckter Position gehalten wird. Dies ist beispielsweise der Fall, wenn eine VND auftritt, wenn also der Verweilschlauch 3 des venösen Zugangs des Patienten aus dem Gefäß des Patienten herausrutscht. In dieser Ruhelage wird der Verweilschlauch 3 durch die durch das Federelement 5 aufgebrachte Kraft bzw. dessen Moment abgeknickt gehalten, wobei sich der Knick an einer Sollknickstelle K des Verweilschlauchs 3 zwischen der ersten Position P1 und der zweiten Position P2, also zwischen dem Basisabschnitt 2 und dem Anschlussabschnitt 6 befindet. Das Federelement 5 ist hierbei vorgespannt und ist mit einer elastischen Rückstellkraft des Verweilschlauchs 3 im Gleichgewicht. Sollte eine VND vorliegen, wirkt die Kraft des Federelements 5 sowohl gegen die Rückstellkraft des Verweilschlauchs 3 als auch gegen einen in dem Verweilschlauch 3 aufgebauten Blutdruck.

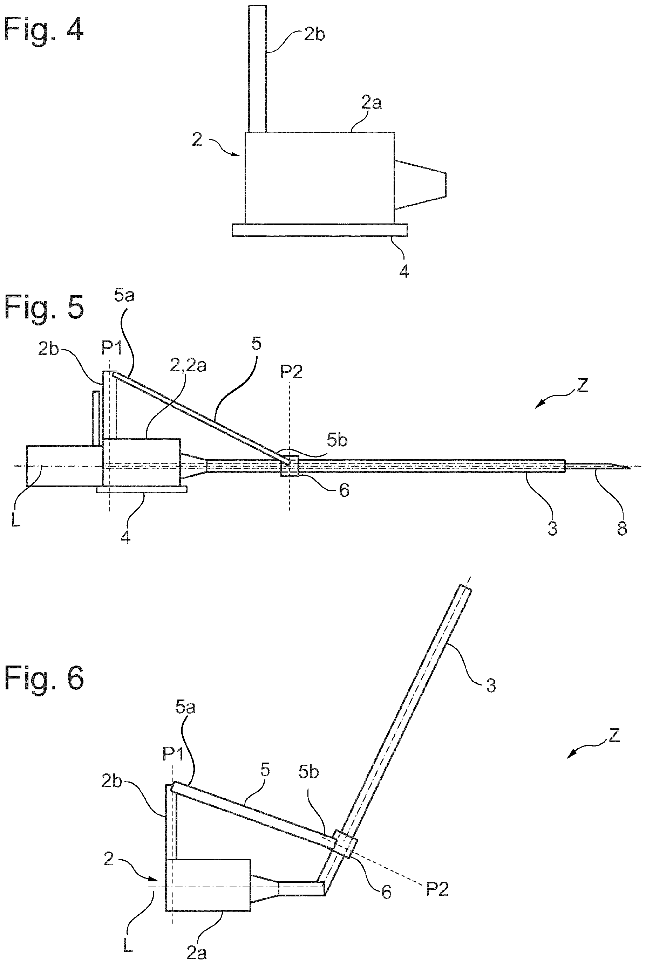

Fig. 4 zeigt einen Teil einer Sperrvorrichtung 1 nach einer zweiten Ausführungsform der Erfindung. Dieses besteht aus einem Basisabschnitt 2, welcher einen Hülsenteil 2a hat, welcher im Wesentlichen dem vorstehend beschriebenen Basisabschnitt 2 nach der ersten Ausführungsform entspricht und ebenfalls einstückig mit einem Befestigungssockel 4 verbunden ist. Darüber hinaus hat der Basisabschnitt 2 nach der zweiten Ausführungsform einen stabförmigen Abstandhalterteil 2b, welcher sich in einer Richtung senkrecht zu der Längsachse L des Verweilschlauchs 3 radial nach außen erstreckt, wobei der Abstandhalterteil 2b und der Hülsenteil 2a einstückig miteinander ausgebildet sind. Der Abstandhalterteil 2b hat ein erstes, radial innenliegendes Ende, an welchem er mit dem Hülsenteil 2a unmittelbar verbunden ist, und ein zweites, radial außen liegendes Ende, welches dazu angepasst ist, ein Federelement 5 zu halten. Das zweite, radial außen liegende Ende weist hierfür beispielsweise eine Kerbe, eine Öse oder Ähnliches auf, in welche das Federelement 5 mit seinem ersten Ende leicht einsetzbar ist.

Fig. 5 zeigt die Zugangskanüle Z nach der zweiten Ausführungsform in einem fertig montierten Zustand, welcher im Wesentlichen, insbesondere in Hinblick auf eine Einsetzbarkeit als ein Zugang für eine Behandlung bzw. dessen Legen und das Zusammenwirken mit einer Einstecknadel 8, der ersten Ausführung nach Fig. 2 entspricht. Im Unterschied zu der ersten Ausführungsform ist in der erfindungsgemäßen Sperrvorrichtung 1 der Zugangskanüle Z nach der ersten Ausführungsform das Federelement 5 als Zugfeder, insbesondere als elastisches Band ausgebildet. Dieses Federelement 5 weist an seinem ersten Ende 5a einen Abschnitt auf, welcher beispielsweise eine Schlaufe, einen Haken oder ein T-Stück formt und welcher dazu angepasst ist, mit dem zweiten, radial außen liegenden Ende des Abstandhalterteils 2b zusammenzuwirken und daran eingesetzt, eingehakt oder um dieses herumgelegt werden kann. Auf diese Weise wird, wie in Fig. 5 dargestellt, das erste Ende 5a des Federelements 5 von dem zweiten, radial außen liegenden Ende des Abstandhalterteils 2b und somit von dem Basisabschnitt 2 gehalten. Die radiale Erstreckung des Abstandhalterteils 2b bzw. eine Länge desselben bestimmt hierbei den Abstand des ersten Endes 5a des Federelements 5 von der Längsachse L des Verweilschlauchs 3.

Ein zweites Ende 5b des Federelements 5 ist mit einem ähnlich oder gleich geformten Abschnitt versehen wie das erste Ende 5a des Federelements 5 und ist dazu angepasst, von einem Anschlussabschnitt 6 der Sperrvorrichtung 1 gehalten zu werden. Der im Wesentlichen hülsenförmig ausgebildete Anschlussabschnitt 6 weist hierfür, ähnlich wie das zweite, radial außen liegende Ende des Abstandhalterteils 2b einen entsprechend angepassten Abschnitt, beispielsweise mit einer Kerbe, einer Öse oder Ähnlichem, auf, durch welchen das zweite Ende 5b des Federelements 5 gehalten wird. Der Anschlussabschnitt 6 ist im Wesentlichen als eine auf den Verweilschlauch 3 aufgeschobene Hülse ausgebildet, welche an einer Position mit einem vorbestimmten Abstand von dem Basisabschnitt 2 gegen Verschieben in Richtung des Basisabschnitts 2 gehalten bzw. gesichert ist, sodass das Federelement 5 zwischen dem Basisabschnitt 2 und dem Anschlussabschnitt 6 gespannt ist. Der Anschlussabschnitt 6 wird dabei durch eine an dem Verweilschlauch 3 ausgebildete Stufe gehalten oder ist an dem Verweilschlauch klebend fixiert. Alternativ hierzu kann auch eine Klemmverbindung eingesetzt werden, der Anschlussabschnitt 6 aus einer simplen Nut oder Rille in dem Verweilschlauch 3 bestehen oder der Anschlussabschnitt 6 direkt an den Verweilschlauch 3 angespritzt bzw. einstückig mit diesem gefertigt werden. Die durch das Federelement 5 auf den Verweilschlauch 3 ausgeübte Kraft ist abhängig und ggf. einstellbar anhand der Elastizität und Länge des Federelements 5, des radialen Abstandes des ersten Endes 5a des Federelements 5 von der Längsachse L des Verweilschlauchs 3 sowie von dem Abstand des Anschlussabschnitts 6 von dem Basisteil 2.

Fig. 6 zeigt die erfindungsgemäße Zugangskanüle Z nach der zweiten Ausführungsform in einer Ruhelage, welche im Wesentlichen der vorstehend bereits in Fig. 3 beschriebenen Darstellung des ersten Ausführungsbeispiels entspricht. Auch hier übt das Federelement 5 eine Kraft mit zumindest einer zu dem Verweilschlauch 3 senkrechten Komponente aus, bzw. beaufschlagt diesen mit einem Moment und hält diesen in einer an einer Sollknickstelle K abgeknickten Lage.

### Liste der Referenzzeichen

- 1: Sperrvorrichtung
- 2: Basisabschnitt
- 2a: Hülsenteil
- 2b: Abstandhalterteil
- 3: Verweilschlauch
- 4: Befestigungssockel oder -pad
- 5: Federelement
- 5a: erstes Ende des Federelements
- 5b: zweites Ende des Federelements
- 6: Anschlussabschnitt
- 7: Aufnahmeloch
- 8: Einstecknadel
- K: Sollknickstelle des Verweilschlauchs
- L: Längsachse des Verweilschlauchs
- P1: erste Position des Verweilschlauchs
- P2: zweite Position des Verweilschlauchs
- Z: Zugangskanüle

## Patentansprüche

1. Zugangskanüle (Z) mit einem Verweilschlauch (3), welcher dazu konfiguriert ist, während einer Behandlung als Zugang zu dienen, einem Befestigungssockel oder einem Befestigungspad (4) zum klebenden Befestigen des Verweilschlauchs (3) an Patientenhaut und einer Sperrvorrichtung (1), welche dazu konfiguriert ist, in einer Ruhelage den Verweilschlauch (3) abgeknickt zu halten,
**dadurch gekennzeichnet, dass** die Sperrvorrichtung (1) ein Federelement (5) aufweist, welches mit einem ersten Ende (5a) an einer ersten Position (P1) des Verweilschlauchs (3) angeordnet ist und welches mit einem zweiten Ende (5b) an einer zweiten Position (P2) an dem Verweilschlauch (3) verbunden oder verbindbar ist, wobei die zweite Position (P2) entlang einer Längsachse (L) des Verweilschlauchs (3) mit einem Abstand von der ersten Position (P1) entfernt ist, und
wobei der Befestigungssockel oder das Befestigungspad (4) an der ersten Position (P1) oder an der zweiten Position (P2) vorgesehen ist.

2. Zugangskanüle (Z) nach Anspruch 1, wobei der Abstand und das Federelement (5) derart eingestellt sind, dass das Federelement (5) gespannt ist, wenn der Verweilschlauch (3) in einer gestreckten Lage gehalten ist.

3. Zugangskanüle (Z) nach Anspruch 1 oder 2, wobei das Federelement (5) an der zweiten Position (P2) eine Federkraft auf den Verweilschlauch (3) ausübt, welche zumindest teilweise senkrecht zu der Längsachse (L) des Verweilschlauchs (3) wirkt, wenn das Federelement (5) gespannt ist.

4. Zugangskanüle (Z) nach einem der Ansprüche 1 bis 3, wobei der Verweilschlauch (3) durch eine Federkraft des Federelements (5) abgeknickt ist, wenn der Verweilschlauch (3) nicht in einer gestreckten Lage gehalten ist.

5. Zugangskanüle (Z) nach Anspruch 4, wobei der Verweilschlauch (3) durch die Federkraft des Federelements (5) an einer Sollknickstelle (K) abgeknickt ist, wenn der Verweilschlauch (3) nicht in einer gestreckten Lage gehalten ist.

6. Zugangskanüle (Z) nach einem der vorstehenden Ansprüche, wobei die Sperrvorrichtung (1) einen Basisabschnitt (2), welcher das erste Ende (5a) des Federelements (5) mit dem Verweilschlauch (3) direkt oder indirekt verbindet oder verbindbar macht, und einen Anschlussabschnitt (6) aufweist, welcher das zweite Ende (5b) des Federelements (5) mit dem Verweilschlauch (3) verbindet oder verbindbar macht.

7. Zugangskanüle (Z) nach Anspruch 6, wobei zumindest der Basisabschnitt (2) und/oder der Anschlussabschnitt (6) mit dem Verweilschlauch oder als ein von dem Verweilschlauch (3) unterschiedliches Bauteil einstückig ausgebildet sind.

8. Zugangskanüle (Z) nach Anspruch 7, wobei die gesamte Sperrvorrichtung (1) inklusive dem Federelement (5) mit dem Verweilschlauch oder als ein von dem Verweilschlauch (3) unterschiedliches Bauteil einstückig ausgebildet ist.

9. Zugangskanüle (Z) nach einem der vorstehenden Ansprüche, wobei das Federelement (5) als Biegefeder ausgebildet ist.

10. Zugangskanüle (Z) nach Anspruch 9, wobei das zweite Ende (5b) der Biegefeder (5) entlang der Längsachse (L) des Verweilschlauchs (3) verschiebbar und somit die zweite Position (P2) des Verweilschlauchs (3) veränderlich ist.

11. Zugangskanüle (Z) nach Anspruch 6, wobei das Federelement (5) als Zugfeder ausgebildet ist.

12. Zugangskanüle (Z) nach Anspruch 11, wobei das Federelement (5) als elastisches Band ausgebildet ist.

13. Zugangskanüle (Z) nach Anspruch 11, wobei die zweite Position (P2) des Verweilschlauchs (3), an welcher das zweite Ende (5b) der Zugfeder (5) gehalten wird, entlang der Längsachse (L) des Verweilschlauchs (3) veränderlich ist und einen vorbestimmten, vorzugsweise durch einen Anschlag definierten, Minimalabstand von der ersten Position (P1) hat oder mit einem vorbestimmten Axialabstand von der ersten Position (P1) an dem Verweilschlauch (3) angebracht ist.

14. Zugangskanüle (Z) nach Anspruch 11, 12 oder 13, wobei der Basisabschnitt (2) derart ausgebildet ist, dass das erste Ende (5a) des als Zugfeder ausgebildeten Federelements (5) an dem Basisabschnitt (2) mit einem vorbestimmten radialen Abstand von der Längsachse (L) des Verweilschlauchs (3) verbunden oder verbindbar ist.

15. Zugangskanüle (Z) nach Anspruch 1, **dadurch gekennzeichnet, dass** der Verweilschlauch (3) an der ersten Position (P1) einen Befestigungssockel oder ein Befestigungspad (4) aufweist, das zur vorzugsweise klebenden Fixierung des Verweilschlauchs (3) an einer Patientenoberfläche, vorzugsweise Patientenhaut ausgebildet ist und an dem das Federelement (5) an dessen erstem Ende (5a) fixiert oder gehalten ist, und der Verweilschlauch (3) an der zweiten Position (P2) ein Anschlusselement oder einen Anschlussabschnitt (6) aufweist, an dem das Federelement (5) an dessen zweitem Ende (5b) fixiert oder gehalten ist, wobei der Verweilschlauch (3) axial zwischen der ersten und zweiten Position (P1, P2) mit einer Soll-Knickstelle (K) ausgebildet ist und das Federelement (5) entweder als Zug- oder Druckfeder, vorzugsweise in Form eines elastischen Bands oder einer Schraubenfeder, oder als Biegefeder, vorzugsweise in Form einer Blattfeder oder eines Federbalkens, ausgebildet ist, die zwischen der ersten und zweiten Position (P1, P2) derart eingebaut und/oder ausgerichtet ist, dass das Federelement (5) in einer gespannten Lage außerhalb der Ruhelage eine Kraft auf den Verweilschlauch (3) an der zweiten Position (P2) ausübt, die zumindest teilweise senkrecht zur Längsachse (L) des Verweilschlauchs (3) wirkt, um diesen aus einer wenig oder nicht geknickten Stellung in die abgeknickte Stellung vorzuspannen.

## Claims

1. An access cannula (Z) comprising a dwell tube (3) configured to serve as an access during treatment, a fixing base or a fixing pad (4) configured for fixing of the dwell tube (3) to a patient's surface and a blocking device (1) configured to maintain the dwell tube (3) kinked in an idle position,
**characterized in that** the blocking device (1) includes a spring element (5) which is arranged with a first end (5a) at a first position (P1) of the dwell tube (3) and which is connected or connectable with a second end (5b) at a second position (P2) on the dwell tube (3), wherein the second position (P2) is distanced along a longitudinal axis (L) of the dwell tube (3) at a distance from the first position (P1), and
wherein the a fixing base or the fixing pad (4) is provided at the first position (P1) or at the second position (P2).

2. The access cannula (Z) according to claim 1, wherein the distance and the spring element (5) are adjusted so that the spring element (5) is tensioned when the dwell tube (3) is maintained in a stretched position.

3. The access cannula (Z) according to claim 1 or 2, wherein at the second position (P2), the spring element (5) exerts a spring force upon the dwell tube (3), said spring force acting at least partly perpendicular to the longitudinal axis (L) of the dwell tube (3) when the spring element (5) is tensioned.

4. The access cannula (Z) according to one of claims 1 to 3, wherein the dwell tube (3) is kinked by a spring force of the spring element (5) when the dwell tube (3) is not maintained in a stretched position.

5. The access cannula (Z) according to claim 4, wherein the dwell tube (3) is kinked at a predetermined kink point (K) by the spring force of the spring element (5) when the dwell tube (3) is not maintained in a stretched position.

6. The access cannula (Z) according to one of the preceding claims, wherein the blocking device (1) includes a base portion (2) which directly or indirectly connects or is connectable the first end (5a) of the spring element (5) to the dwell tube (3), and includes a fitting portion (6) which connects or is connectable the second end (5b) of the spring element (5) to the dwell tube (3).

7. The access cannula (Z) according to claim 6, wherein at least the base portion (2) and/or the fitting portion (6) are formed integrally with the dwell tube (3) or as a component different from the dwell tube (3).

8. The access cannula (Z) according to claim 7, wherein the entire blocking device (1) including the spring element (5) is formed integrally with the dwell tube (3) or integrally as a component different from the dwell tube (3).

9. The access cannula (Z) according to one of the preceding claims, wherein the spring element (5) is in the form of a bending spring.

10. The access cannula (Z) according to claim 9, wherein the second end (5b) of the bending spring (5) is movable along the longitudinal axis (L) of the dwell tube (3) and thus the second position (P2) of the dwell tube (3) is variable.

11. The access cannula (Z) according to according to claim 6, wherein the spring element (5) is in the form of a tension spring.

12. Access cannula (Z) according to claim 11, wherein the spring element (5) is configured as an elastic strip.

13. The access cannula (Z) according to claim 11, wherein the second position (P2) of the dwell tube (3) at which the second end (5b) of the tension spring (5) is held is variable along the longitudinal axis (L) of the dwell tube (3) and has a predetermined minimum distance from the first position (P1), preferably defined by a stop, or is arranged at the dwell tube (3) at a predetermined axial distance from the first position (P1).

14. The access cannula (Z) according to claim 11, 12 or 13, wherein the base portion (2) is configured so that the first end (5a) of the spring element (5) in the form of a tension spring is connected or connectable at the base portion (2) at a predetermined radial distance from the longitudinal axis (L) of the dwell tube (3).

15. The access cannula (Z) according to claim 1, **characterized in that** the dwell tube (3) at the first position (P1) includes a fixing base or a fixing pad (4) configured for preferably adhesive fixing of the dwell tube (3) to a patient's surface, preferably patient skin, and on which the spring element (5) is fixed or held at the first end (5a) thereof, and the dwell tube (3) at the second position (P2) includes a fitting element or a fitting portion (6) at which the spring element (5) is fixed or held at the second end (5b) thereof, wherein the dwell tube (3) is formed axially between the first and second positions (P1, P2) with a predetermined kink point (K) and the spring element (5) is configured either as a tension or compression spring, preferably as an elastic strip or a coil spring, or as a bending spring, preferably in the form of a leaf spring or a spring beam, mounted and/or aligned between the first and the second position (P1, P2) so that in a stretched position outside the idle position the spring element (5) exerts force upon the dwell tube (3) at the second position (P2), said force acting at least partly perpendicular to the longitudinal axis (L) of the dwell tube (3) so as to pretension the latter from a slightly or not kinked position into the kinked position.

## Revendications

1. Canule d'accès (Z) avec un tuyau à demeure (3), lequel est configuré pour servir d'accès pendant un traitement, un socle de fixation ou un tampon de fixation (4) pour la fixation adhésive du tuyau à demeure (3) sur la peau d'un patient et un dispositif de blocage (1), lequel est configuré pour maintenir le tuyau à demeure (3) fléchi dans une position de repos,
**caractérisée en ce que** le dispositif de blocage (1) présente un élément ressort (5), lequel est disposé avec une première extrémité (5a) au niveau d'une première position (P1) du tuyau à demeure (3) et lequel est relié ou peut être relié à une deuxième extrémité (5b) au niveau d'une deuxième position (P2) sur le tuyau à demeure (3), dans laquelle la deuxième position (P2) est espacée de la première position (P1) avec un écart le long d'un axe longitudinal (L) du tuyau à demeure (3), et
dans laquelle le socle de fixation ou le tampon de fixation (4) est prévu au niveau de la première position (P1) ou au niveau de la deuxième position (P2).

2. Canule d'accès (Z) selon la revendication 1, dans laquelle l'écart et l'élément ressort (5) sont réglés de telle sorte que l'élément ressort (5) est tendu lorsque le tuyau à demeure (3) est maintenu dans une position étirée.

3. Canule d'accès (Z) selon la revendication 1 ou 2, dans laquelle l'élément ressort (5) au niveau de la deuxième position (P2) exerce une force élastique sur le tuyau à demeure (3), laquelle agit au moins en partie perpendiculairement à l'axe longitudinal (L) du tuyau à demeure (3) lorsque l'élément ressort (5) est tendu.

4. Canule d'accès (Z) selon l'une quelconque des revendications 1 à 3, dans laquelle le tuyau à demeure (3) est fléchi par une force élastique de l'élément ressort (5), lorsque le tuyau à demeure (3) n'est pas maintenu dans une position étendue.

5. Canule d'accès (Z) selon la revendication 4, dans laquelle le tuyau à demeure (3) est fléchi par la force élastique de l'élément ressort (5) au niveau d'un point de flexion théorique (K), lorsque le tuyau à demeure (3) n'est pas maintenu dans une position étendue.

6. Canule d'accès (Z) selon l'une quelconque des revendications précédentes, dans laquelle le dispositif de blocage (1) présente une partie de base (2), laquelle relie ou rend reliable directement ou indirectement la première extrémité (5a) de l'élément ressort (5) au tuyau à demeure (3), et présente une partie de raccordement (6), laquelle relie ou rend reliable la deuxième extrémité (5b) de l'élément ressort (5) au tuyau à demeure (3).

7. Canule d'accès (Z) selon la revendication 6, dans laquelle au moins la partie de base (2) et/ou la partie de raccordement (6) sont réalisées d'une seule pièce avec le tuyau à demeure ou sous la forme d'une pièce différente du tuyau à demeure (3).

8. Canule d'accès (Z) selon la revendication 7, dans laquelle tout le dispositif de blocage (1) y compris l'élément ressort (5) est réalisé avec le tuyau à demeure ou sous la forme d'une pièce différente du tuyau à demeure (3).

9. Canule d'accès (Z) selon l'une quelconque des revendications précédentes, dans laquelle l'élément ressort (5) est réalisé sous la forme d'un ressort de flexion.

10. Canule d'accès (Z) selon la revendication 9, dans laquelle la deuxième extrémité (5b) du ressort de flexion (5) est déplaçable le long de l'axe longitudinal (L) du tuyau à demeure (3) et donc la deuxième position (P2) du tuyau à demeure (3) est variable.

11. Canule d'accès (Z) selon la revendication 6, dans laquelle l'élément ressort (5) est réalisé sous la forme d'un ressort de traction.

12. Canule d'accès (Z) selon la revendication 11, dans laquelle l'élément ressort (5) est réalisé sous la forme d'une bande élastique.

13. Canule d'accès (Z) selon la revendication 11, dans laquelle la deuxième position (P2) du tuyau à demeure (3), au niveau de laquelle la deuxième extrémité (5b) du ressort de traction (5) est maintenue, est variable le long de l'axe longitudinal (L) du tuyau à demeure (3) et a un écart minimal prédéfini, défini de préférence par une butée, par rapport à la première position (P1) ou est monté sur le tuyau à demeure (3) avec un écart axial prédéfini par rapport à la première position (P1) .

14. Canule d'accès (Z) selon la revendication 11, 12 ou 13, dans laquelle la partie de base (2) est réalisée de telle sorte que la première extrémité (5a) de l'élément ressort (5) réalisé sous la forme d'un ressort de traction est reliée ou peut être reliée sur la partie de base (2) avec un écart radial prédéfini par rapport à l'axe longitudinal (L) du tuyau à demeure (3).

15. Canule d'accès (Z) selon la revendication 1, **caractérisée en ce que** le tuyau à demeure (3) présente au niveau de la première position (P1) un socle de fixation ou un tampon de fixation (4), qui est réalisé pour la fixation de préférence adhésive du tuyau à demeure (3) sur une surface d'un patient, de préférence peau d'un patient et sur lequel l'élément ressort (5) est fixé ou maintenu au niveau de la première extrémité (5a) de celui-ci, et le tuyau à demeure (3) présente au niveau de la deuxième position (P2) un élément de raccordement ou une partie de raccordement (6), sur lequel(laquelle) l'élément ressort (5) est fixé ou maintenu sur la deuxième extrémité (5b) de celui-ci, dans laquelle le tuyau à demeure (3) est réalisé axialement entre la première et deuxième position (P1, P2) avec un point de fléchissement théorique (K) et l'élément ressort (5) est réalisé sous la forme d'un ressort soit de traction soit de compression, de préférence sous forme d'une bande élastique ou d'un ressort hélicoïdal, ou sous la forme d'un ressort de flexion, de préférence sous forme d'un ressort à lames ou d'une barre à ressort, qui est installé(e) et/ou orienté(e) entre la première et deuxième position (P1, P2) de telle sorte que l'élément ressort (5) dans une position tendue hors de la position de repos exerce une force sur le tuyau à demeure (3) au niveau de la deuxième position (P2), qui agit au moins en partie perpendiculairement à l'axe longitudinal (L) du tuyau à demeure (3), afin de précontraindre celui-ci à partir d'une position peu ou non fléchie dans la position fléchie.
